# EUROPEAN PATENT SPECIFICATION

(11) **EP 1 384 476 B1**
(45) Date of publication and mention of the grant of the patent: **02.03.2005**
(21) Application number: 02714232.2
(22) Date of filing: 21.03.2002
(51) Int. Cl.: A61K 31/415, A61K 31/381, A61P 11/06, A61P 11/08, A61P 11/14

(54) **AZOLYLCARBYNOL DERIVATIVES OF ARYL (OR HETEROARYL) FOR THE TREATMENT OF RESPIRATORY DISEASES**
AZOLYLCARBYNOL-DERIVATIVE VON ARYL (ODER HETEROARYL) ZUR BEHANDLUNG VON ATEMWEGSERKRANKUNGEN
DERIVES D'ARYLE (OU HETEROARYLE) AZOLYLCARBINOLES POUR LE TRAITEMENT DE MALADIES RESPIRATOIRES

(30) Priority: 06.04.2001 ES 200100817
(43) Date of publication of application: 28.01.2004
(73) Proprietor: LABORATORIOS DEL DR. ESTEVE, S.A., 08041 Barcelona (ES)
(72) Inventor: MERCE-VIDAL, Ramon, E-08041 Barcelona (ES); ANDALUZ-MATERO, Blas, E-08041 Barcelona (ES); FRIGOLA-CONSTANSA, Jordi, E-08041 Barcelona (ES)
(74) Representative: Carpintero Lopez, Francisco
(86) International application number: PCT/ES2002/000136
(87) International publication number: WO 2002/080908

(56) References cited:
- ES-A- 2 137 136
- ES-A- 2 150 353
- ES-A- 2 150 378

## Description

### Area of the Invention

The present invention concerns the use of aryl (or heteroaryl) azolylcarbinole derivatives of general formula I and their physiologically acceptable salts, as drugs in human and/or veterinary therapeutics for the treatment of respiratory diseases especially for the treatment of cough, bronchitis, chronic obstructive pulmonary diseases, allergic rhinitis and asthma.

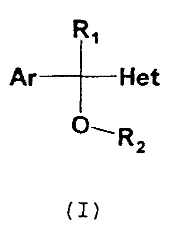

### Background of the Invention

Coughing is a physiological defence mechanism to eliminate foreign bodies and excess bronchial secretion in the respiratory airways, although it is also a common symptom of a wide range of respiratory diseases. The antitussives currently available in the therapeutic arsenal are effective but, in addition to other side effects, are sedatives, which limits their use. Therefore, although the antitussives available are widely used, the search for new and better-tolerated products in still on going.

The cough reflex is triggered by activation of the rapid adaptation dilatation receptors (or irritation receptors) in the larynx, trachea and proximal bronchi, slow adaptation dilatation receptors and fibre C receptors, that are found on the walls of the bronchial respiratory airways. Therefore, a peripheral antitussive mechanism would inhibit the "inputs" of the afferent nerve fibres or activation of the sensorial receptors of the respiratory airways. The availability of a new, clinically effective and non-narcotic drug would, therefore, represent a significant improvement in current cough treatment.

In our patents EP 289380 and WO 99/52525 we have described carbinole derivatives of general formula (I) 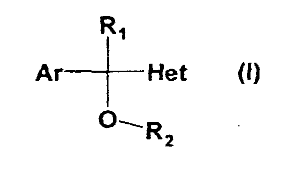 in which Ar represents a benzene ring or a thiophene ring, with or without substitutions, R₁ represents a hydrogen atom or a lower alkyl group from C₁ to C₄; R₂ represents a dialkylaminoalkyl radical or azaheterocyclylalkyl and Het represents a substituted azole, and its physiologically acceptable salts.

In our patents WO 97/20817, WO 99/02500, WO 99/07684 and WO 99/52525 we have also described several procedures for the preparation of enantiomerically pure compounds of general formula (I).

We have also discovered now that general formula (I) compounds, and their physiologically acceptable salts, are especially useful to elaborate drugs, in human and/or veterinary therapeutics to cure or relieve respiratory diseases, especially for the treatment of cough, bronchitis, chronic obstructive pulmonary diseases, allergic rhinitis and asthma.

### Detailed description of the invention

The present invention concerns the use of aryl (or heteroaryl) azolylcarbinole derivatives of general formula (I) 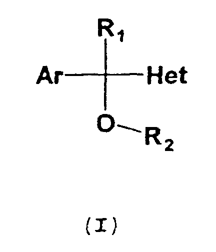 in which
Ar represents a phenyl radical or a thienyl radical, without substitutions or with 1,2 or 3 equal or different substituents, selected from the group comprised by fluoride, chloride, bromide, methyl, trifluoromethyl and methoxy;
R₁ represents a hydrogen atom of a lower alkyl group from C₁ to C₄;
R₂ represents a dialkyl radical (C₁-C₄)aminoalkyl (C₂-C₃), or azaheterocyclylalkyl (C₂-C₃); and
Het represents an azole, i.e. an aromatic nitrogenated heterocycle of five members that contains from one to three nitrogen atoms, not substituted or optionally substituted by 1 or 2 equal or different substituents selected from the group comprised by fluoride, chloride, bromide and methyl;
Or one of its physiologically acceptable salts,
In the elaboration of a drug for the treatment of respiratory diseases especially for the treatment of cough, bronchitis, chronic obstructive pulmonary disease, allergic rhinitis and asthma, in mammals, including man.

The term "lower alkyl group from C₁ to C₄" represents a straight or branched chain radical derived from a saturated carbohydrate with 1 to 4 carbon atoms, for example methyl, ethyl, propyl, isopropyl, butyl, isobutyl, *sec*-butyl and *terc*-butyl.

The term "dialkyl(C₁-C₄)aminoalkyl (C₂-C₃), or azaheterocyclylalkyl (C₂-C₃)" represents an alkyl radical of two or three carbon atoms bound to a dialkyl (C₁-C₄) amine or a cyclic amine, such as for example dimethylaminoethyl, dimethylaminopropyl, dimethylaminopropyl, diethylaminoethyl, piperidinylethyl, morpholinylpropyl, pirrolidinylalkyl, etc.

Examples for illustration of compounds encompassed by the present invention include:
(±)-5-{α-[2-(dimethylamine)ethoxy]benzyl}-1-methyl-1*H*-pirazole.
(±)-5-{α-[2-(dimethylamine)ethoxy]benzyl}-1-methyl-1*H*-pirazole citrate.
(+)-5-{α-[2-(dimethylamine)ethoxy]benzyl}-1-methyl-1*H*-pirazole
(-)-5-{α-[2-(dimethylamine)ethoxy]benzyl}-1-methyl-1*H*-pirazole
(+)-5-{α-[2-(dimethylamine)ethoxy]benzyl}-1-methyl-1*H*-pirazole citrate.
(-)-5-{α-[2-(dimethylamine)ethoxy]benzyl}-2-methyl-1*H*-pirazole citrate.
(±)-5-{α-[2-(dimethylamine)ethoxy]-2-thienylmethyl}-1-methyl-1*H*-pirazole.
(±)-5-{α-[2-(dimethylamine)ethoxy]-2-thienylmethyl}-1-methyl-1*H*-pirazole citrate.
(+)-5-{α-[2-(dimethylamine)ethoxy]-2-thienylmethyl}-1-methyl-1*H*-pirazole.
(-)-5-{α-[2-(dimethylamine)ethoxy]-2-thienylmethyl}-1-methyl-1*H*-pirazole.
(+)-5-{α-[2-(dimethylamine)ethoxy]-2-thienylmethyl}-1-methyl-1*H*-pirazole citrate.
- (-)-5-{α-[2-(dimethylamine)ethoxy]-2-thienylmethyl}-1-methyl-1*H*-pirazole citrate.

The compounds of general formula (I) can be synthesised according to the procedures described in the patents EP 289380 or WO 99/52525. The compounds of general formula (I) have a stereogenic centre and the invention concerns both the utilisation of a pure enantiomere and of a mixture of enantiomeres. The enantiomeres can be prepared by some of the procedures described in our patents WO 97/20817, WO 99/02500, WO 99/07684 or WO 99/52525.

In the present invention, the antitussive activity of the general formula (I) compounds has been demonstrated which are also unaccompanied by any sedative effect. To do this, the antitussive activity has been studied in guinea-pigs, simultaneously studying the possible sedative effects at the doses used in this study. The method used was the one described by Boura [Boura ALA, Green AF, Saunders IA (1970), An antitussive test using guinea-pigs. *Br. J. Pharmacol*., 39, 225P], con modificaciones más recientes [Adcock JJ, Schneider C, Smith TW (1988), Effects of codeine, morphine a novel opioid pentapeptide BW443C, on cough, nociception and ventilation in the unanaesthetized guinea-pig. *Br. J. Pharmacol*., 93 : 93-100; Clay TP, Thompson MA (1985), Irritant induced cough as a model of intrapulmonary airway reactivity. *Lung*, 163 : 183-191; Forsberg K, Karlsson J-A, Theodorsson E; Lundberg JM; Persson CGA (1988), Cough and bronchoconstriction mediated by capsaicin-sensitive sensory neurons in the guinea-pig. *Pulm Pharmacol*, 1: 33-39; Gallico L, Borghi A, Dalla Rosa C, Ceserani R, Tognella S (1994), Moguisteine: a novel peripheral non-narcotic antitussive drug. *Br. J. Pharmacol*, 112 : 795-800].

The following examples describe some of the properties that form the object of the invention for (±)-5-{α-[2-(dimethylamine)ethoxy]benzyl}-1-methyl-1*H*-pirazole citrate of formula

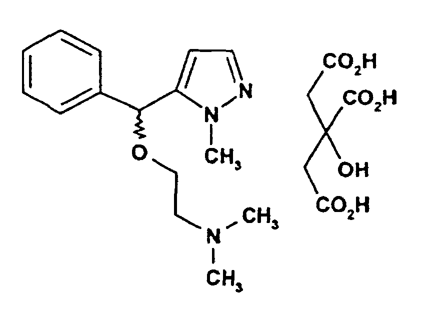

The following examples that describe some pharmacological trials are merely illustrative and the application of the invention is by no means limited to these.

### Antitussive activity of (±)-5-{α-[2-(dimethylamine)ethoxy]benzyl}-1-methyl-1H-pirazole citrate

The guinea-pigs (250-400 g) were deprived of water and food 30 minutes before any intervention. The guinea-pigs, neither ligated nor anaesthetised, were individually placed in a perspex box (20 x 12 x 14 cm.) and left for an accommodation period of 15 minutes. After this period, the animals were exposed to an aerosol of an aqueous solution of citric acid (7.5 % p/v, 0.39M). This concentration of citric acid is the minimum concentration capable of producing the maximum number of coughs during the observation period (Forsberg et al., 1988). The aerosol was generated for 2.5 minutes using an ultrasound nebulizer (De Vilbiss, Ultraneb 99; mean particle size 0.9 µm, with a flow of 0.5 ml/min.). The observation period with the animals exposed to the nebulized citric acid in the box was 12.5 minutes (2.5 minutes of aerosol production, followed by an additional 10 minutes). During this period, the animals were observed continuously, the number of coughs was counted and the baseline value was taken as control. Only the guinea-pigs that coughed between 15 and 30 times during the observation period were selected for the study. On the day after the selection the animals were randomly treated with the vehicle or with the study product thirty minutes before being exposed again to the citric acid aerosol. The antitussive activity was assessed for each guinea-pig as the reduction in the number of coughs compared to their previously recorded values. On the other hand, the animals were observed from the time of administration of the product until the end of the experiment for possible sedative effects.

The table shows the results obtained with (±)-5-{α-[2-(dimethylamine)ethoxy]benzyl}-1-methyl-1*H*-pirazole, where the product can be observed to have a good antitussive activity which is perfectly dose-related. The product was administered at doses of 10, 40 and 100 mg/kg, via ip and does not produce any sedative effect in treated animals.

### Antitussive activity of (±)-5-{α-[2-(imethylamine)ethoxy]benzyl}-1-methyl-1H-pirazole citrate against cough induced by citric acid aerosol in guinea-pig.

| Product | N | N° of Coughs±SEM | % Inhibition | SD-50 |
|---|---|---|---|---|
| Vehicle | 10 | 13.8 ± 0.9 | - | |
| Product | | | | |
| (10 mg/kg, ip) | 10 | 8.7 ± 0.7 | 37% | |
| Product | | | | |
| (40 mg/kg,ip) | 10 | 4.3 ± 0.5 | 71% | 17.6 mg/kg,ip |
| Product | | | | |
| (100 mg/kg,ip) | 10 | 2.8 ± 0.4 | 80% | |

The results obtained show that the products of the patent are clear antitussives, since (±)-5-{α-[2-(dimethylamine)ethoxy]benzyl}-1-methyl-1*H*-pirazole citrate has a SD-50 of 17.6 mg/kg, ip, unaccompanied by any sedative effect since at the maximum dose used 100 mg/kg, ip, there was no sedative effect.

Taking into account their good pharmacodynamic properties, aryl (or heteroaryl)azolylcarbinole derivatives, according to the invention, can be satisfactorily used in human and animal therapeutics to cure or relieve respiratory diseases.

In human therapeutics, the administrative dose for the compounds of this invention depends on the severity of the condition to be treated. Normally this is between 50 and 400 mg/day. The compounds of the invention will be administered, for example, in capsule form, tablets or injectable solutions.

Next, we indicate, as an example, specific pharmaceutical formulae of the compounds that form part of the present invention.

### Example of injectable formula (im/iv):

| | |
|---|---|
| (±)-5-{α-[2-(dimethylamine)ethoxy] | |
| benzyl}-1-methyl-1*H*-pirazole citrate | 50 mg |
| Sodium hydroxide 0.1 M | pH 6 |
| Water for injection | 1 ml |

### Example of formula per tablet

| | |
|---|---|
| (±)-5-{α-[2-(dimethylamine)ethoxy] | |
| benzyl)-1-methyl-1*H*-pirazole citrate | 400 mg |
| Sodium Croscaramelose (Ac-Di-Sol) | 32 mg |
| Coloidal silica dioxide (Aerosil 200) | 8 mg |
| Magnesium stearate, NF | 16 mg |
| Povidone K-30 | 40 mg |
| Microcrystalline Cellulose (Avicel PH-102) | 146 mg |
| Monohydrate Lactose (Farmatose 200M) | 158 mg |
| Total | 800 mg |

### Example of formula per capsule

| | |
|---|---|
| (±)-5-{α-[2-(dimethylamine)ethoxy] | |
| benzyl}-1-methyl-1*H*-pirazole citrate | 200.0 mg |
| Coloidal silica dioxide | 0.8 mg |
| Magnesium stearate | 2.4 mg |
| Lactose | 276.8 mg |
| Total | 480 mg |

## Claims

1. Use of an aryl (or heteroaryl)azolylcarbinole derivative of general formula (I) 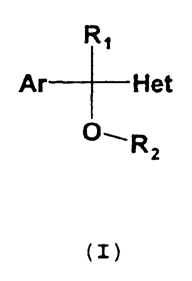 in which
Ar represents a phenyl radical or a thienyl radical, not substituted or optionally substituted by 1, 2 or 3 equal or different sustituents, selected from the group comprised by fluoride, chloride, bromide, methyl, trifluoromethyl and methoxy;
R₁ represents a hydrogen atom or a lower alkyl group from C₁ to C₄;
R₂ represents a dialkyl radical (C₁-C₄)aminoalkyl (C₂-C₃), or azaheterocyclylalkyl (C₂-C₃) ; and
Het represents a five member nitrogenated aromatic heterocycle containing one to three nitrogen atoms, without substitutions or optionally substituted by 1 or 2 equal or different substituents selected from the group comprised by fluoride, chloride, bromide and methyl;
or one of its physiologically acceptable salts,
in the elaboration of a drug to treat respiratory diseases especially to treat cough, bronchitis, chronic obstructive pulmonary respiratory diseases, allergic rhinitis and asthma, in mammals, including man.

2. The use, according to Claim 1, of a compound of general formula (I), in which R₁ is selected from among a hydrogen atom or from the group comprised of methyl, ethyl, propyl, isopropyl, butyl, isobutyl, sec-butyl and terc-butyl, in the production of a drug to treat respiratory diseases especially for the treatment of cough, bronchitis, chronic obstructive pulmonary diseases, allergic rhinitis and asthma, in mammals, including man.

3. The use, according to Claim 1, of a compound of general formula (I), in which R₂ is selected from the group comprised by dimethylaminoethyl, dimethylaminopropyl, diethylaminoethyl, piperidinylethyl, morpholinylpropyl and pirrolidinylethyl, in the production of a drug to treat respiratory diseases especially for the treatment of cough, bronchitis, chronic obstructive pulmonary diseases, allergic rhinitis and asthma, in mammals, including man.

4. The use, according to Claim 1, of a compound of general formula (I) selected from the group comprised by:
(±)-5-{α-[2-(dimethylamine)ethoxy]benzyl}-1-methyl-1*H*-pirazole.
(±)-5-{α-[2-(dimethylamine)ethoxy]benzyl}-1-methyl-1*H*-pirazole citrate.
(+)-5-{α-[2-(dimethylamine)ethoxy]benzyl}-1-methyl-1*H*-pirazole.
(-)-5-{α-[2-(dimethylamine)ethoxy]benzyl}-1-methyl-1*H*-pirazole.
(+)-5-{α-[2-(dimethylamine)ethoxy]benzyl}-1-methyl-1*H*-pirazole citrate.
(-)-5-{α-[2-(dimethylamine)ethoxy]benzyl}-1-methyl-1*H*-pirazole citrate.
(±)-5-{α-[2-(dimethylamine)ethoxy]-2-thienylmethyl}-1-methyl-1*H*-pirazole.
(±)-5-{α-[2-(dimethylamine)ethoxy]-2-thienylmethyl}-1-methyl-1*H*-pirazole citrate.
(+)-5-{α-[2-(dimethylamine)ethoxy]-2-thienylmethyl}-1-methyl-1*H*-pirazole.
(-)-5-{α-[2-(dimethylamine)ethoxy]-2-thienylmethyl}-1-methyl-1*H*-pirazole.
(+)-5-{α-[2-(dimethylamine)ethoxy]-2-thienylmethyl}-1-methyl-1*H*-pirazole citrate.
(-)-5-{α-[2-(dimethylamine)ethoxy]-2-thienylmethyl}-1-methyl-1*H*-pirazole citrate.
in the production of a drug to treat respiratory diseases especially to treat cough, bronchitis, chronic obstructive pulmonary diseases, allergic rhinitis and asthma, in mammals, including man.

## Patentansprüche

1. Verwendung einer Abweichung der allgemeinen Formel (I) des Aryl-(oder Heteroaryl-) Azolylkarbinols 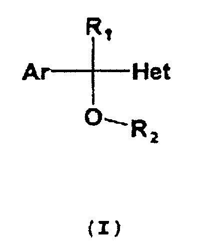 bei dem
Ar ein Phenylradikal oder ein Thienylradikal ist, welches nicht oder optional durch 1, 2 oder 3 gleiche oder unterschiedliche Austauschstoffe ersetzt wird, die aus einer Gruppe von Fluorid, Chlorid, Brom, Methyl, Trifluormethyl und Methoxyl gewählt werden;
R1 stellt ein Wasserstoffatom oder eine niedere Alkylgruppe von C1 bis C4 dar;
R² stellt ein Dialkylradikal (C1 bis C4), Aminoalkyl (C²-C³) oder Azaheterocyclylalkyl (C²-C³) und
Het stellt einen fünfgliedrigen stickstoffhaltigen aromatischen Heterocyclus mit einem bis drei Stickstoffatomen, ohne Austauschstoffe oder optional durch 1, 2 oder 3 gleiche oder unterschiedliche Austauschstoffe ersetzt, die aus einer Gruppe von Fluorid, Chlorid, Brom, Methyl oder eines ihrer physiologisch geeigneten Salze bestehen,
dies in der Ausarbeitung eines Antibiotikums zur Behandlung von Bronchopneumopathie und besonders zur Behandlung von Husten, Bronchitis, chronisch obstruktive Lungenerkrankungen, Stockschnupfen und Asthma bei Säugetieren einschließlich Menschen.

2. Verwendung nach Anspruch 1, eines Teils der allgemeinen Formel (I), in der R1 aus einem Wasserstoffatom oder einer Gruppe aus Methyl, Äthyl, Propyl, Isopropyl, Butyl, Isobutyl, Sec-Butyl und Terc-Butyl besteht
dies in der Ausarbeitung eines Antibiotikums zur Behandlung von Bronchopneumopathie und besonders zur Behandlung von Husten, Bronchitis, chronisch obstruktive Lungenerkrankungen, allergischer Rhinopathie und Asthma bei Säugetieren einschließlich Menschen.

3. Verwendung nach Anspruch 1 eines Teils der allgemeinen Formel (I), in der R² aus einer Gruppe aus Dimethylaminoäthyl, Dimethylaminoproyl, Diethylaminoethyl, Piperidinylethyl, Morpholinylpropyl und Pirrolidinylethyl besteht
dies in der Ausarbeitung eines Antibiotikums zur Behandlung von Bronchopneumopathie und besonders zur Behandlung von Husten, Bronchitis, chronisch obstruktive Lungenerkrankungen, allergischer Rhinopathie und Asthma bei Säugetieren einschließlich Menschen.

4. Verwendung nach Anspruch 1 eines Teils der allgemeinen Formel (I), gewählt aus einer aus folgendem bestehenden Gruppe:
(+/-)-5-{a-2-[Dimethylamino]Ethoxy}Benzyl}-1-Methyl-1*H*-Pirazol.
(+/-)-5-{a-2-[Dimethylamino]Ethoxy}Benzyl}-1-Methyl-1*H*-Pirazol-Citrat
(+)-5-{a-2-[Dimethylamino]Ethoxy}Benzyl}-1-Methyl-1*H*-Pirazol
(-)-5-{a-2-[Dimethylamino]Ethoxy}Benzyl}-1-Methyl-1*H*-Pirazo
(+)-5-{a-2-[Dimethylamino]Ethoxy}Benzyl}-1-Methyl-1*H*-Pirazol-Citrat
(-/-)-5-{a-2-[Dimethylamino]Ethoxy}Benzyl}-1-Methyl-1*H*-Pirazol-Citrat
(+/-)-5-{a-2-[Dimethylamino)Ethoxy]-2-Thienylmethyl}-1-Methyl-1*H*
Pirazol
+/-)-5-{a-2-[Dimethylamino)Ethoxy]-2-Thienylmethyl}-1-Methyl-1*H*-Pirazol-Citrat
(+)-5-{a-[2-(Dimethylamino)Ethoxy]-2-Thienylmethyl}-1-Methyl-1*H*-Pirazol
(-)-5-{a-[2-(Dimethylamino)Ethoxy]-2-Thienylmethyl}-1-Methyl-1*H*-Pirazol
(+)-5-{a-[2-(Dimethylamino)Ethoxy]-2-Thienylmethyl}-1-Methyl-1*H*-Pirazol-Citrat
(-)-5-{a-[2-(Dimethylamino)Ethoxy]-2-Thienylmethyl}-1-Methyl-1*H*-Pirazol-Citrat
dies in der Ausarbeitung eines Antibiotikums zur Behandlung von Bronchopneumopathie und besonders zur Behandlung von Husten, Bronchitis, chronischen Lungenatembeschwerden, allergischer Rhinopathie und Asthma bei Säugetieren einschließlich Menschen.

## Revendications

1. Utilisation d'un dérivé d'aryl ou (hétéroaryl)azolylcarbinole de formule générale (I) 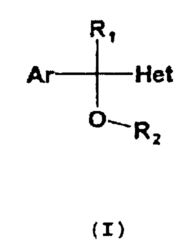 dans laquelle :
Ar représente un radical phényle ou un radical thiényle, non substitué ou optionnellement substitué par 1, 2 ou 3 substituants identiques ou différents, sélectionnés parmi le groupe constitué par fluorure, chlorure, bromure, méthyle, trifluorométhyle et méthoxy.
R₁ représente un atome d'hydrogène ou un groupe alkyle inférieur de C₁ à C₄;
R₂ représente un radical dialkyle (C₁-C₄), aminoalkyle (C₂-C₃) ou azahétérocyclyalkyle (C₂-C₃) ; et
Het représente un hétérocycle aromatique azoté de cinq membres contenant de un à trois atomes de nitrogène, sans substitution ou étant optionnellement substitués par 1 ou 2 substituants identiques ou différents sélectionnés parmi le groupe constitué par du fluorure, chlorure, bromure et méthyle ; ou un de ses sels physiologiquement acceptables, dans l'élaboration d'un médicament pour traiter des maladies respiratoires spécialement pour traiter la toux, la bronchite, les maladies respiratoires pulmonaires obstructives chroniques, la rhinite allergique et l'asthme, chez les mammifères y compris l'homme.

2. Utilisation, selon la revendication 1, d'un composé de formule générale (I), dans laquelle R₁ est sélectionné parmi un atome d'hydrogène ou parmi le groupe constitué par méthyle, éthyle, propyle, isopropyle, butyle, isobutyle, sec-butyle et *tert*-butyle, dans la production d'un médicament pour traiter des maladies respiratoires spécialement pour le traitement de la toux, la bronchite, les maladies pulmonaires obstructives chroniques, la rhinite allergique et l'asthme, chez les mammifères y compris l'homme.

3. Utilisation, selon la revendication 1, d'un composé de formule générale (I), dans laquelle R₂ est sélectionné parmi le groupe constitué par diméthylaminoéthyle, diméthylaminopropyle, diéthylaminoéthyle, pipéridinyléthyle, morpholinylpropyle, et pirrolidinyléthyle, dans la production d'un médicament pour traiter des maladies respiratoires spécialement pour le traitement de la toux, la bronchite, les maladies pulmonaires obstructives chroniques, la rhinite allergique et l'asthme, chez les mammifères y compris l'homme.

4. Utilisation, selon la revendication 1, d'un composé de formule générale (1) sélectionné parmi le groupe constitué par :
(±)-5-{α-[2-(diméthylamine)éthoxy]benzyl}-1-méthyl-1*H*-pirazole. Citrate de (±)-5-{α-[2-(diméthylamine)éthoxy]benzyl}-1-méthyl-1*H*-pirazole
(+)-5-{α-[2-(diméthylamine)éthoxy]benzyl}-1-méthyl-1*H*-pirazole.
(-)-5-{α-[2-(diméthylamine)éthoxy]benzyl}-1-méthyl-1*H*-pirazole.
Citrate de (+)-5-{α-[2-(diméthylamine)éthoxy]benzyl}-1-méthyl-1*H*-pirazole.
Citrate de (-)-5-{α-[2-(diméthylamine)éthoxy]benzyl}-1-méthyl-1*H*-pirazole.
(±)-5-{α-[2-(diméthylamine)éthoxy]-2-thiénylméthyl}-1-methyl-1*H*-pirazole.
Citrate de (±)-5-{α-[2-(diméthylamine)éthoxy]-2-thiénylméthyl}-1-methyl-1*H*-pirazole.
(+)-5-{α-[2-(diméthylamine)éthoxy]-2-thiénylméthyl}-1-methyl-1*H*-pirazole.
(-)-5-{α-[2-(diméthylamine)éthoxy]-2-thiénylméthyl}-1-methyl-1*H*-pirazole.
Citrate de (+)-5-{α-[2-(diméthylamine)éthoxy]-2-thiénylméthyl}-1-methyl-1*H*-pirazole.
Citrate de (-)-5-{α-[2-(diméthylamine)éthoxy]-2-thiénylméthyl}-1-methyl-1*H*-pirazole.
dans la production d'un médicament pour traiter des maladies respiratoires spécialement pour traiter la toux, la bronchite, les maladies pulmonaires obstructives chroniques, la rhinite allergique et l'asthme, chez les mammifères y compris l'homme.
